# EUROPEAN PATENT APPLICATION

(11) **EP 1 566 198 A2**
(43) Date of publication of application: **24.08.2005**
(21) Application number: 04468027.0
(22) Date of filing: 21.12.2004
(51) Int. Cl.: A61N 1/32

(54) **Hand-help apparatus for skin treatment**

(30) Priority: 22.12.2003 SI 200300315
(71) Applicant: Bremed d.o.o. Breginj, 5223 Breginj (SI)
(72) Inventor: Moise, Franco, 24070 Mossa (GO) (IT)
(74) Representative: Kraljic, Janez

(57) **Abstract**

The hand-help apparatus for skin treatment by present invention is a device designed in a way to be used and controlled only by one hand. On its surface it has at least two mutually separated and electrically insulated electrically conductible areas, preferentially metallic electrodes. Device is to be placed with its first electrode 1 on the part of skin where the iontoforesis and/or galvanic foresis procedure is performed. Its second electrode 2 is placed on the casing of device 4 so that it is in contact with the hand of a person on who is performing the iontoforesis and/or galvanic foresis procedure on own body. On device there is switch 6 for program selection and switch 9 by which the polarity of electrodes 1 and 2 can be changed. The said hand-help apparatus for skin treatment by this invention for its operation uses standard exchangeable batteries.

## Description

The field of invention.

The invention is referred to electric hand-help apparatus for skin treatment.

The state of technique.

The iontoforesis method is known. It is penetrating the curing substances of medicine or cosmetic preparations into the body trough the skin with the help of galvanic current. With the opposite operation which is by exchanging the electrode polarities the galvanic foresis procedure is performed. Its effect is cleaning of the skin with the help of galvanic current. So far existing devices for performing of iontoforesis and/or galvanic foresis are being table-type devices which have electrical connections for skin care with galvanic current. In this regard are needed two electrical cables on which two electrical conductible plates - electrodes are connected. The electrical connection can also be made of two mutually separated and insulated electrodes on the same conducting cable. The first electrode is placed on the same part of skin on which the iontoforesis procedure is performed and/or galvanic foresis. The second electrode is in contact with the hand of person on which the iontoforesis and/or galvanic foresis procedure is performed. In this way the electric circuit is connected. These devices are connected to external energy source, usually on the network voltage. Because of their size and relatively disturbing and inconvenient cables these devices are generally used in the beauty saloons and physiotherapeutic ordinations.

### Description of the invention.

Hand-help apparatus for skin treatment according to present invention is a device which is designed and made in a way that it can be used and controlled with one hand. On its surface it has at least two mutually separated and electrically insulated electrically conductible areas, preferentially metallic electrodes. Device should be with its first electrode 1 placed on the part of skin where the iontoforesis and/or galvanic foresis procedure is performed. Its second electrode 2 has to be placed on the casing of device 4 so that it is in contact with the hand of a person on which the iontoforesis and/or galvanic foresis procedure is performed. On the casing 4 of device the switch 6 for program selection is placed and the switch 9 by which the polarity of electrodes 1 and 2 can be exchanged even during the operation itself. The said hand-help apparatus for skin treatment by this invention uses standard exchangeable batteries.

The invention will be additionally described in with the help of pictures which shows:

### Description of pictures:

- Picture 1: represents one possible variant of hand-help apparatus for skin treatment by present invention,
- Picture 2: represents second possible variant of hand-help apparatus for skin treatment by present invention,
- Picture 3: shows scheme of electronic circuit that is used for the device operation.

Hand-help apparatus for skin treatment is composed of casing 4 in which there is 9 V exchangeable alkaline battery type 6LR61 and electronic circuit that is shown on picture 3.

On the front part the hand-help apparatus for skin treatment has its first electrode 1 and behind it in direction of casing 4 the lightening surface of red pulse light diode (CLL) 3. At the side of casing there are conductible surfaces which represent second electrode 2 for touching the hand of a person who is using on own self the device for performing procedures of iontoforesis and/or galvanic foresis. On the upper part of the casing 4 is placed switch 5 with ON/OFF function of the device. Switch 6 for the performing program selection and switch 9 for exchanging the polarity of electrodes 1 and 2. On casing there are also LED indicators which show the state of device. Red indicator 7 warns user about ON either OFF state of device and of battery exhaustion. 5 white LED indicators 8 on the casing 4 of device denote the chosen program.

As it is evident from the picture the hand-help apparatus for skin treatment by present invention in contrariety to existent devices for performing procedures of iontoforesis and/or galvanic foresis does not need any connecting cable between the unit and the person who is performing the above described procedure on one's own body, neither does not need electrically conductible plates which would be connected to those cables.

The hand-help apparatus for skin treatment by present invention has namely two mutually electrically insulated electrically conductible surfaces on its casing. When using the device one conductive surface which is represented by the first electrode 1 is placed on skin's surface where iontoforesis and/or galvanic foresis procedure takes place. With the other conductive surface which is represented by the second electrode 1 the constant contact with the hand of a person who on one's own self performs chosen procedures of iontoforesis and/or galvanic foresis.

Additional advantage of the hand-help apparatus for skin treatment by present invention is the characteristic that with procedure of iontoforesis and/or galvanic foresis simultaneously is performed also procedure of photo massage with the red pulse light generated by red pulse light diode (CLL).

Electrical circuit for low levels of electrical current which is needed for performing of iontoforesis and/or galvanic foresis procedure is connected by turning ON and setting the device into its operating position. It is by placing the first electrode 1 on the contact with place on the body where the application of iontoforesis and/or galvanic foresis performed and with restoring the contact between the second electrode 2 and the the hand by which this hand-help apparatus for skin treatment is used and controlled. In this way first cable for connection of the first electrode 1 on the place where we apply the procedures of iontoforesis and/or galvanic foresis does not exist, because the first electrode 1 is in this case the component part of the device itself. Also the second cable for the second electrode is needless. It is replaced by the hand of a person who is performing on one's own body the procedure of iontoforesis and/or galvanic foresis when he/she is by the hand in direct contact with that part of the hand-help apparatus for skin treatment by present invention which represents the second electrode 2.

Special advancement of the hand-help apparatus for skin treatment by present invention is that the polarity of the first 1 and the second 2 electrode can be changed by the switch 9 placed on the device's casing 4. By that advantage fast and easy exchange of direction can be made during performing procedures of iontoforesis and/or galvanic foresis and there is no need of interruption at the skin care procedures and removing the device from its operating position. In the cosmetic usage we achieve by switching over very fast and effective application of cosmetic preparations with iontoforesis and intensive cleaning of skin with galvanic foresis. With hand-help apparatus for skin treatment by present invention by procedure of iontoferosis the curing substances of medicine in spite of their positive or negative polarity are penetrating quickly and effectively. The polarity can be also changed whenever during the process simply by pressing the switch 9 on the casing of device and therefore there is no need to replace or even physical change of electrodes or cables.

This kind of device at which the polarity of electrodes is possible to change quickly and easily is especially convenient at procedures for curing the mycogenic and similar diseases. The user which is in this case the patient will with hand-help apparatus for skin treatment in a simple and quick way apply anti mycotic preparations everywhere in spite of where he/she is and without using remised, troublesome and knotty external cables with electrodes which are making applying of preparation on skin difficult. Besides that, devices with cables and electrodes demand suitable place for their usage. Simple and effective in the same way is the hand-help apparatus for skin treatment at penetrating by iontoferosis of those curing substances of those medicines which are used for curing of muscular pains, cramps, joint affections, etc.

Both electrodes 1, 2 on the casing of device 4 for skin care are preferentially made of extra treated metal material. They are firmly fixed on the casing 4 and are simultaneously a mechanical component of this handy device. Cleaning of such electrodes 1, 2 is therefore simple. Electrodes can be simply wiped up with proper detergent and handkerchief. Electrodes are sterilised by that. By taking into consideration all the hygienic norms the hand-help apparatus for skin treatment can therefore be safely used unlimited time. It can be used by many different patients.

Electronic circuit scheme which controls the present hand-help apparatus for skin treatment is shown on the picture 3. Electronic circuit is made on the basis of microcontroller Microchip PIC16F630. Microcontroller is programmed in such a way that it together with electronic circuit performs 10 programs of iontoforesis or galvanic foresis. 5 programs at which the first electrode 1 which is on place of the activity is negative and the second electrode 2 which is on the casing of device 4 is positive, and another 5 programs with opposite polarity of electrodes 1, 2. For skin stimulation at performing of programs collaborates also the red pulse light diode (CLL) with the light which has wavelength 630 nm, frequency 21 Hz and the radiation angle of 18 degrees.

Iontoferesis programs which we perform with the above described hand-help apparatus for skin treatment at which the first electrode 1 is negative and the second electrode 2 is positive are defined with the following characteristics:
Program 1:
   Electrical current of 150 µA flows from the first electrode in direction towards skin at the place where we perform the procedure.
   Type of electrical current: direct electrical current, which is generated by 12 V direct electrical voltage, covered with unsymmetrical triangle impulses of 5 mV operating voltage and frequency 12 kHz.
   Operation of red laser light diode (CLL) has frequency 21 Hz.
Program 2:
   Electrical current of 250 µA flows from the first electrode in direction towards skin at the place where we perform the procedure.
   Type of electrical current: direct electrical current, which is generated by 20 V direct electrical voltage, covered with unsymmetrical triangle impulses of 10 mV operating voltage and frequency 12 kHz.
   Operation of red laser light diode (CLL) has frequency 21 Hz.
Program 3:
   Electrical current of 450 µA flows from the first electrode in direction towards skin at the place where we perform the procedure.
   Type of electrical current: direct electrical current, which is generated by 33 V direct electrical voltage, covered with unsymmetrical triangle impulses of 15 mV operating voltage and frequency 12 kHz.
   Operation of red laser light diode (CLL) has frequency 21 Hz.
Program 4:
   Electrical current of 150 µA flows from the first electrode in direction towards skin at the place where we perform the procedure.
   Type of electrical current: direct electrical current, which is generated by 12 V direct electrical voltage, covered with unsymmetrical triangle impulses of 5 mV operating voltage and frequency 12 kHz. Said electrical current in this program lasts 1 sec then follows 1 sec of break. Electrical current and the break are exchanging all the time during operation.
   Operation of red laser light diode (CLL) has frequency 21 Hz.
Program 5:
   Electrical current of 250 µA flows from the first electrode in direction towards skin at the place where we perform the procedure.
   Type of electrical current: direct electrical current, which is generated by 20 V direct electrical voltage, covered with unsymmetrical triangle impulses of 10 mV operating voltage and frequency 12 kHz. Said electrical current in this program lasts 2 sec then follows 2 sec of break. Electrical current and the break are exchanging all the time during operation.
   Operation of red laser light diode (CLL) has frequency 21 Hz.

Programs from 1 to 5 are the programs for carrying out the procedures of iontoforesis by which effectively penetrate substances of any cosmetic preparation in the skin where the cosmetic substance must include at least 2% of natural non distilled water.

The efficiency of cosmetic products is by use of hand-help apparatus for skin treatment by present invention increased from 30 to 80%, depending on the kind of skin and on the mixture in cosmetic preparation. We achieve that high efficiency of cosmetic preparation because of the ionoforesis and the effect of red pulse light (CLL) controlled by programs from 1 to 5. Both contribute to much more effective penetration of cosmetic preparation into the skin in compare to normal method or with manual procedure of cosmetic preparation's application.

Programs where the first electrode 1 is positive and the second electrode 2 is negative are defined with the following characteristics:
Program 6:
   Electrical current of 150 µA flows from the skin at the place where we perform the procedure in direction towards first electrode.
   Type of electrical current: direct electrical current, which is generated by 12 V direct electrical voltage, covered with unsymmetrical triangle impulses of 5 mV operating voltage and frequency 12 kHz.
   Operation of red laser light diode (CLL) has frequency 21 Hz.
Program 7:
   Electrical current of 250 µA flows from the skin at the place where we perform the procedure in direction towards first electrode.
   Type of electrical current: direct electrical current, which is generated by 20 V direct electrical voltage, covered with unsymmetrical triangle impulses of 10 mV operating voltage and frequency 12 kHz.
   Operation of red laser light diode (CLL) has frequency 21 Hz.
Program 8:
   Electrical current of 450 µA flows from the skin at the place where we perform the procedure in direction towards first electrode.
   Type of electrical current: direct electrical current, which is generated by 33 V direct electrical voltage, covered with unsymmetrical triangle impulses of 15 mV operating voltage and frequency 12 kHz.
   Operation of red laser light diode (CLL) has frequency 21 Hz.
Program 9:
   Electrical current of 150 µA flows from the skin at the place where we perform the procedure in direction towards first electrode.
   Type of electrical current: direct electrical current, which is generated by 12 V direct electrical voltage, covered with unsymmetrical triangle impulses of 5 mV operating voltage and frequency 12 kHz. Said electrical current in this program lasts 1 sec then follows 1 sec of break. Electrical current and the break are exchanging all the time during operation.
   Operation of red laser light diode (CLL) has frequency 21 Hz.
Program 10:
   Electrical current of 250 µA flows from the skin at the place where we perform the procedure in direction towards first electrode.
   Type of electrical current: direct electrical current, which is generated by 20 V direct electrical voltage, covered with unsymmetrical triangle impulses of 10 mV operating voltage and frequency 12 kHz. Said electrical current in this program lasts 2 sec then follows 2 sec of break. Electrical current and the break are exchanging all the time during operation.
   Operation of red laser light diode (CLL) has frequency 21 Hz.

Programs from 6 to 10 have function of performing galvanic foresis. They are used for accurate skin cleaning.

Efficiency of skin cleaning increases by use of the hand-help apparatus for skin treatment by present invention by using those programs together with additional usage of normal cosmetic detergents or only with the water and neutral soap. Galvanic foresis helps impurities coming out of skin. By procedures of galvanic foresis cleaning effect increases for about 50 to 80% in comparison with traditional manual skin cleaning procedures. At these programs the effect of red pulse light generated by CLL is a simple photo massage of skin.

The hand-help apparatus for skin treatment by present invention has programmed automatic switch off of device in case when it is not in use for certain period. Each program can be interrupted whenever during the operation. The polarity can be also whenever changed with the switch 9.

Operation of the hand-help apparatus for skin treatment by present invention is controlled by microcontroller Microchip PIC16F630. Low power DC-DC converter controlled by the same microcontroller generates required electrical current for iontoforesis and/or galvanic foresis.

Operation of hand-help apparatus for skin treatment by present invention allows energy from exchangeable battery of 6LR61 type (alkaline 9 V battery) inside of casing 4. Maximum consumption of electrical current including with consumption of red pulse light and LED indicators is less than 25 mA.

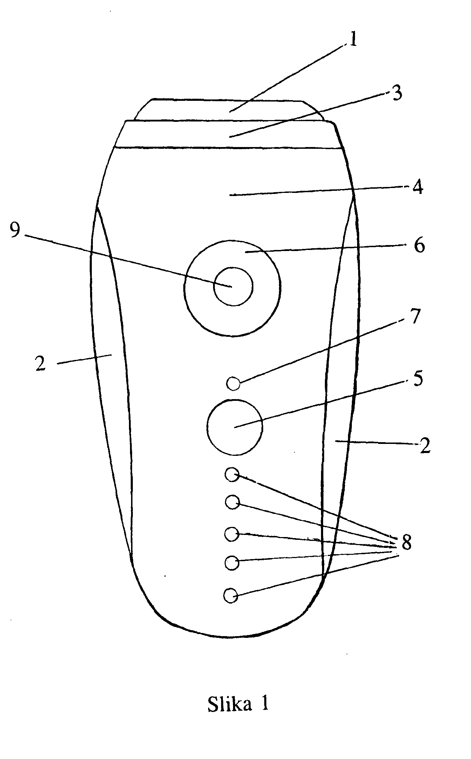

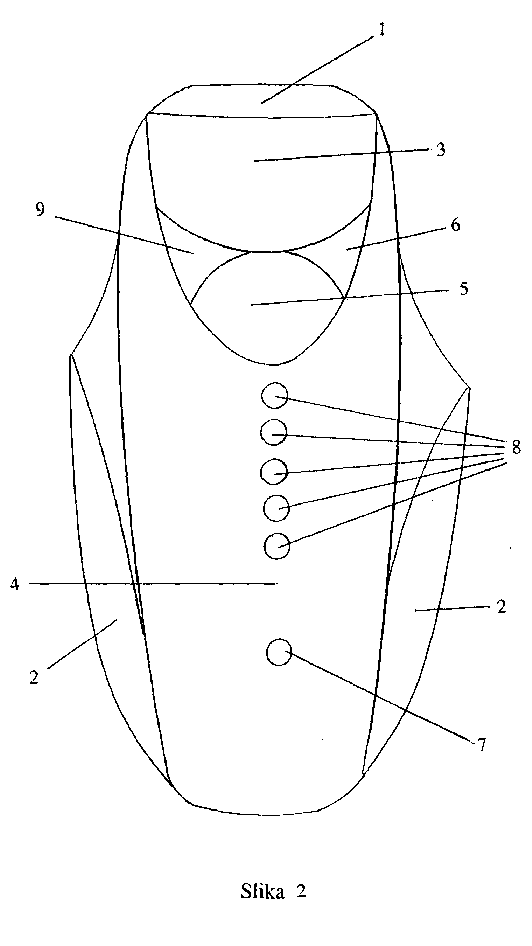

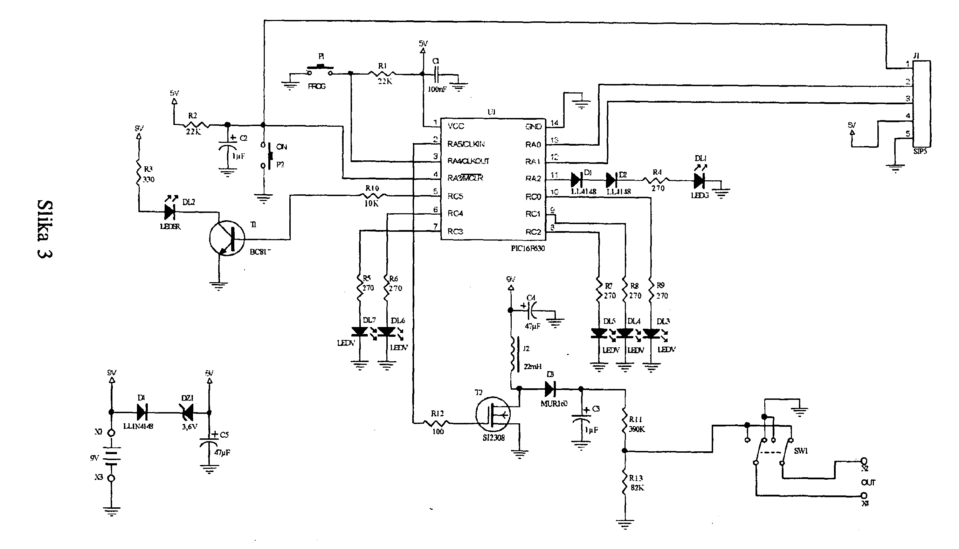

## Claims

1. The hand-help apparatus for skin treatment for performing of iontoforesis and/or galvanic foresis procedures comprises the casing (4) suitable to hold it in hand and which has ON/OFF switch (5) on the upper side of the casing, switch (6) for performing program selection, and LED signal indicators which denote ON and OFF of device, the battery exhaustion and selected program, in the casing (4) is electronic circuit on the basis of microcontroller and exchangeable battery, **characterised in that** on the casing (4) are placed two mutually electrically insulated and electrically conductible areas, electrodes (1, 2) on which the electronic circuit supplies 12 - 33 V of direct (DC) electrical voltage covered by unsymmetrical triangle impulses of 5 - 15 mV operating voltage of 12 kHz frequency, by which during performing of iontoforesis and/or galvanic foresis procedures electrical current is 150 to 450 µA from the first (1) through the body to the second electrode (2) or in the opposite direction.

2. The hand-help apparatus for skin treatment according to the claim 1 **characterised in that** further comprises on the casing switch (9) for exchanging the polarity of the first (1) electrode and the second (2) electrode on the casing (4) of device and by that during performing of iontoforesis and/or galvanic foresis procedures direction of electric current through the body is changed.

3. The hand-help apparatus for skin treatment according to the claim 1 or 2 **characterised in that** further comprises beside the first electrode (1) on casing (4) built in laser red pulse light (3) which allows that with procedure of iontoforesis and/or galvanic foresis simultaneously are also performed procedures of photo massage with the red pulse light.
